# EUROPÄISCHE PATENTSCHRIFT

(11) **EP 0 676 408 B1**
(45) Veröffentlichungstag und Bekanntmachung des Hinweises auf die Patenterteilung: **14.11.2001**
(21) Anmeldenummer: 95104624.2
(22) Anmeldetag: 29.03.1995
(51) Int. Cl.: C07H 17/08, C07H 15/16, A61K 9/16, A61K 9/14

(54) **Verfahren zur Herstellung eines antibakteriellen Wirkstoffes und dessen Verwendung**
Process for the production of antibacterial agents and their use
Procédé de préparation d'agents antibactériens et leur utilisation

(30) Priorität: 08.04.1994 SE 9401169
(43) Veröffentlichungstag der Anmeldung: 11.10.1995
(73) Patentinhaber: CORIMED GmbH, D-64807 Dieburg (DE)
(72) Erfinder: Bauer, Hans Jörg, D-55234 Flomborn (DE)
(74) Vertreter: Wagner, Karl Heinz

(56) Entgegenhaltungen:
- EP-A- 0 277 008
- DD-A- 275 398
- PATENT ABSTRACTS OF JAPAN vol. 008 no. 134 (C-230) ,21.Juni 1984 & JP-A-59 044310 (NIPPON KAYAKU KK) 12.März 1984,

## Beschreibung

Die vorliegende Erfindung bezieht sich auf ein Verfahren zur Herstellung eines antibakteriellen Wirkstoffes und dessen Verwendung.

In der Wundinfektionsbekämpfung stehen die Behandlungen bereits infizierter Wundgebiete im Vordergrund. Die breite Verwendung von Antibiotika, die seit den 40er Jahren under der Entdeckung des Penizillins mehr und mehr an Bedeutung gewonnen haben, rückte diese Behandlungsmethoden in den Vordergrund.

Die rasche Entwicklung von Operationstechniken, Operationshilfsstoffen under Operationsräume in Richtung Asepsis und Sterilität und das Bestreben zur Reduzierung der Früh- und Spätinfektionen bewirkte eine Entwicklung, die die prophylaktischen Maßnahmen zur Verhinderung von Infektionen bei operativen Eingriffen maßgeblich positiv beeinflußt haben.

In der medizinischen Anwendung sind die antibakteriellen Versorgungen der Patienten in Verbindung mit der Verwendung von Fixationshilfsstoffen- oder Mitteln, oder zur lokalen Therapie seit der breiten Anwendung antibiotikahaltiger Knochenzemente, adäquaten Trägern oder anderen resorbierbaren oder nicht resorbierbaren medizinischen Biomaterialien als Stand der Technik zu betrachten.

Die in der DD 275 398 A1 beschriebene Methode zur Herstellung feinverteilter pharmazeutischer Wirkstoffe ist auch als Stand der Technik zu bewerten. In diesem Verfahren wird ein fein gemahlener Wirkstoff in einer Lösung des Wirkstoffes mit verringerter niedrigen Löslichkeit umkristallisiert. Die Verarbeitung zum Endprodukt wird dabei nicht näher beschrieben, sondern lediglich als galenischer Prozess gekennzeichnet.

Jedoch konnten die Nachteile der bisher bekannten Wirkstoffe und/oder Träger und deren Kombinationen in Bezug auf die nicht optimale Wirkstoffreisetzung in vorbestimmter Zeit und Menge nicht behoben werden, was zu den Weiterentwicklungen auf diesem Forschungsgebiet geführt hat.

Aufgabe der Erfindung war es nun, die Verfügbarkeit des oder der Wirkstoffe in oder mit Biomaterialien - oder auch allein - sowohl der Verfügbarkeitszeit nach als auch der Wirkstoffmenge pro Zeitenheit zu optimieren und die praktische Anwendbarkeit in Herstellung und Applikation wesentlich zu verbessern.

Erfindungsbemäß wird die Aufgabe dahingehend gelöst, daß Wirkstoffe - vorzugsweise handelsübliche Antibiotika, wie z.B. Gentamicin, Clindamycin, Erythromicin, Vancomicin, oder allgemein Aminoglykoside, Cepholosporine, Penizillin, Gyrasehemmer oder Andere, derart behandelt werden, daß aus einer Lösung eines oder mehreren Wirkstoffen ein Mono- oder Copräzipitat in kristalliner und/oder amorpher Form durch Umkristallisation hergestellt wird und daß Lösungsmittel ganz oder teilweise entfernt wird. Der bei dieser Methode hergestellte Kuchen aus Wirkstoff wird daran anschließend auf das angestrebte Anwendungsgebiet hin zerkleinert, wobei die Korngrößenverteilung einer gestauchten gauß'schen Glockenkurve mit abgeflachten Plateau angestrebt wird. Im Idealfall stellt diese Krongrößenverteilung ein Trapez mit steilen Flanken dar.

Dieses Verfahren ermöglicht die erfindungsgemäß optimale Freisetzung des oder der Wirkstoffe in exakt vorbestimmbarer Zeit und Menge allein oder aus einem vorgegebenen Trägermaterial.

Abbildung I zeigt eine derartige Korngrößenverteilung.

Abbildung II zeigt das schematische Freisetzungsverhalten der in Abbildung I dargestellten Korngrößenverteilung.

Durch die quantitative Kombination verschiedener Korngrößenverteilungen ist es nun möglich, die initiale Anfangsdosis in Art und Menge einzustellen, so daß sich zum Beispiel an eine Freisetzung mit schneller und hoher Anfangskoncentration eines Wirkstoffes eine protrahierte Wirkstoffreisetzung mit breitem Plateau anschließt.

Abbildung III zeigt schematisch ein solches Verhalten.

Gleiches ist natürlich auch bei der relevanten Beeinflussung der Koncentrationsabnahmegeschwindigkeit mit zeitlicher Terminierung der Freisetzung möglich.

Eine weitere erfindungsgemäße Methode zur Herstellung und Verwendung eines antibakteriellen Wirkstoffes ist nachfolgend beschrieben.

Ein wie oben beschrieben hergestellter umkristallisierter Wirkstoff oder Wirkstoffkombination wird mit einem weiteren Wirkstoff umhüllt, indem er durch einen feinen Nebel des zweiten Wirkstoffes entweder hindurchgeblasen wird, im gravimetrischen Fall durch den Wirkstoffnebel 2 hindurchgeleitet wird oder in oder auf einem Wirbelbett mit dem Wirkstoff 2 umhüllt wird. Daran anschließend erfolgt die Kristallisation des zweiten Wirkstoffe, der Umhüllung.

Da die ursprünglichen Ausgangswirkstoffe sehr leicht löslich sind, erfolgt ein inniger Verbund der beiden Wirkstoffe, da der erste Wirkstoff während der Beschichtung leicht angelöst wird und so eine Verbindungsschicht von erstem und zweitem Wirkstoff entstehen kann.

Die so hergestellte Wirkstoffkombination in Kornform hat durch die weitestgehende Umhüllung des ersten Wirkstoffes mit dem zweiten Wirkstoff die Besonderheit im Freisetzungsverhalten, daß zuerst der Wirkstoff 2 freigesetzt wird, dann durch die Verbindungsschicht hervorgerufen eine Kombination von Wirkstoff 2 und 1 und erst dann die Freisetzung des ersten Wirkstoffes einsetzt. Kern oder Hülle können dabei auch durch andere Stoffe, die nicht Wirkstoffe sind, wie z.B. Polymere, Keramiken oder sonstige, ausgewechselt werden.

Besonders hervorzuheben an dieser erfinderischen Methode der Wirkstoffherstellung ist die Herstellung von Wirkstofformkörpern.

Zur Herstellung von Wirkstofformkörpern werden eine oder mehrere Wirkstoffgrundsubstanzen gelöst und in weitestgehend definierter Schichtdicke in eine Kristallisationsbehälter umkristallisiert. Der Wirkstoffkuchen werden nach dem Entformen von möglicherweise vorhandenen Zonen unterschiedlicher Dichte am Boden und Decke befreit und daran anschließend in die gewünschte Form geschnitten. Bei der Herstellung ist auf die Temperaturführung und die Druckverhältnisse zu achten, da sonst die inhomogenen (Dichte) Schichten stark an Volumen zunehmen. Wesentlich and dieser Methode ist, daß es so möglich ist eine größere Menge antibakteriellen Wirkstoffes gezielt zu plazieren ohne ihn bereits während der Applikation im gesamten Wundgebiet zu verteilen und so seine lokale Wirkung zu schmälern.

Mit der letztgenannten Herstellungsmethode können schließlich auch im Voraus festgelegte geometrische Formen hergestellt werden, indem die Kristallisationsgefäße bereits der Form entsprechen und die antibakteriellen Wirkstofformen in diesen umkristallisiert werden. Die Herstellung von Formkörpern, wie z.B. Stangen, Blättchen, Kugeln, Quadern, Zylindern etc. oder auch in Form einer Kette, oder auch in einer räumlichen Anordnung wie einem Netzt oder einem dreidimensionalem Gitter ist ebenfalls realisierbar.

In Bezug auf Kombinationspräparate ist die extrem gute Mischbarkeit der Wirkstofflösungen mit Füllstoffen ein erfindungsgenäßes Verfahren.

In die Wirkstofflösung wird der resorbierbare oder nichtresorbierbare pulver- oder körnerförmige Füllstoff, z.B. Calciumphosphate, Zirkonoxide, Aluminiumoxide, Polymere o.A. in chemisch gefällter Form, vernetzt oder in keramischer Form homogen eingerührt. Die dabei entstehende Paste, deren, Viskosität eingestellt werden kann, kann sowohl in dieser pastösen Form direkt appliziert werden, z.B. mit Hilfe einer Spritze, oder nach dem vorher beschriebenen Verfahren zu Formkörpern oder Granulat verarbeitet werden.

Als weitere Methode wird erfindungsbemäss die Kombination mit Gips hervorgehoben. Die Anmischung erfolgt wie bei den Kombinationspräparaten, wobei hier die in vivo Härtung des Gipses für ein temporäres Implantat sorgt. Selbstverständlich können aber auch Formkörper vorgefertigt werden und als Implantate eingesetzt werden.

Allen Methoden gemeinsam ist die verbesserte Freisetzung des/der Wirkstoffe, da die reine initiale Löslichkeit mit Zunahme des Umkristallisationsgrades, bzw. mit Zunahme des/der Füllstoffe abnimmt und so das Korn, bzw. der Formkörper nicht bereits bei der Applikation aufgelöst wird.

Exemplarische Beispiele für die vorher beschribenen Wirkstofformen und deren Herstellung:

### Beispiel 1:

1 kg Clindamycin wird unter definierten thermischen Bedingungen (z.B. 35°C) in 800 ml reinem Wasser unter kontinuierlichem Rühren vollständig gelöst. Die erhaltene visköse Lösung wird in einer wohldefinierten Schichtdicke (z.B. 1,3 cm) bis zur Mahltrockene unter Vakuum (z.B. 150 mbar absolut) gleichmässig getrocknet. Die Clindamycinkuchen werden ggf. von inhomogenen Dichteschichten befreit und in einem Korngrössenselektrierendem Mahlverfahren (z.B. Sichtermühle, oder erst Mahlung dann Klassierung) auf eine trapezförmige Korngrößenverteilung von 195 *µ*m bis 215 *µ*m zerkleinert och selektiert. Das erhaltene Clindamicinkorn findet seinen Einsatz z.B. als antibakterieller Bestandteil von Knochenzemenzen, Kollagenvliesen, Polymeren wie Lactid/Glycolid etc.

### Beispiel 2:

1 kg Erythromicin wird wie in Beispiel 1 beschrieben gelöst, jedoch mit 900 ml reinem Wasser. In die Lösung wird 1,4 kg feinstes gefälltes Hydroxylapatitpulver, welches mit 15% strahlungsarmen Zirkoniumoxid dotiert ist, homogen eingemischt und die entstandene Paste wird mit einer Schichtdicke von z.B. 1 cm auf der Kristallisationsform blasenfrei ausgestrichen. Danach wird wie im Beispiel 1 beschriebenen Verfahren vorgegangen.

### Beispiel 3:

Wie Beispiel 2, wobei der Wirkstoffkuchen nicht gemahlen wird, sondern in Blöcke von z.B. 5 mm x 5 mm x 10 mm zerschnitten wird.

### Beispiel 4:

Wie Beispiel 2, wobei die Paste auf einer Kristallisationsform 1 in aneinandergereihte kegelige Hohlformen 2, welche untereinander durch eine Linie 3, in die ein medizinischer Faden oder Draht 4 eingelegt ist - siehe Abbildung IV - eingestrichen wird und nach der Umkristallisation gemeinsam entformt werden, so daß eine Kette aus Wirkstoffkörpern 5 entsteht.

Weitere Beispiele lassen sich aus den vorherigen Beschreibungen ableiten.

Das hergestellte Material kann als Zuschlagstoff für Formkörper für die medizinische Anwendung, auch für die äusseren medizinischen Anwendung, verwendet werden. Das hergestellte Material kann auch als Zuschlagstoff für medizinische Wachse, Kleber (z.B. medizinische Zemente), Siegel oder andere Trägermateriale, verwendet werden.

Ausserdem kann das hergestellte Material als Streukorn zur lokalen antibakteriellen Therapie, als Beschichtungsmaterial für Implantate oder zur Einbringung in dafür vorgesehene Kavitäten in den Implantaten und in der Orthopädie, Chirurgie und Kieferchirurgie, verwendet werden.

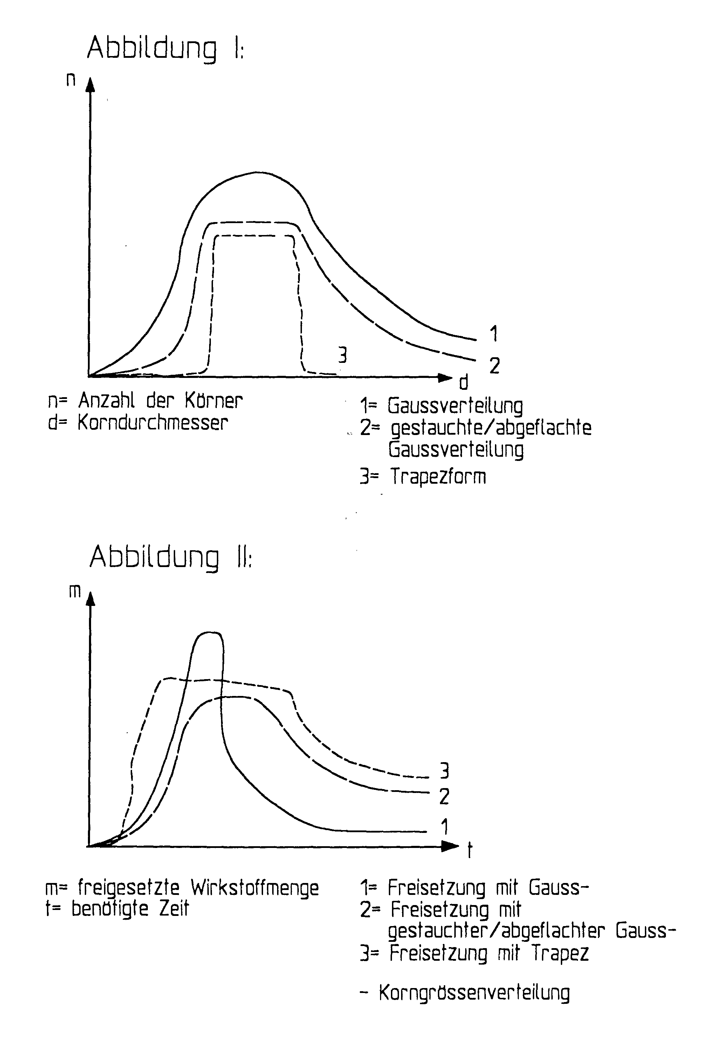

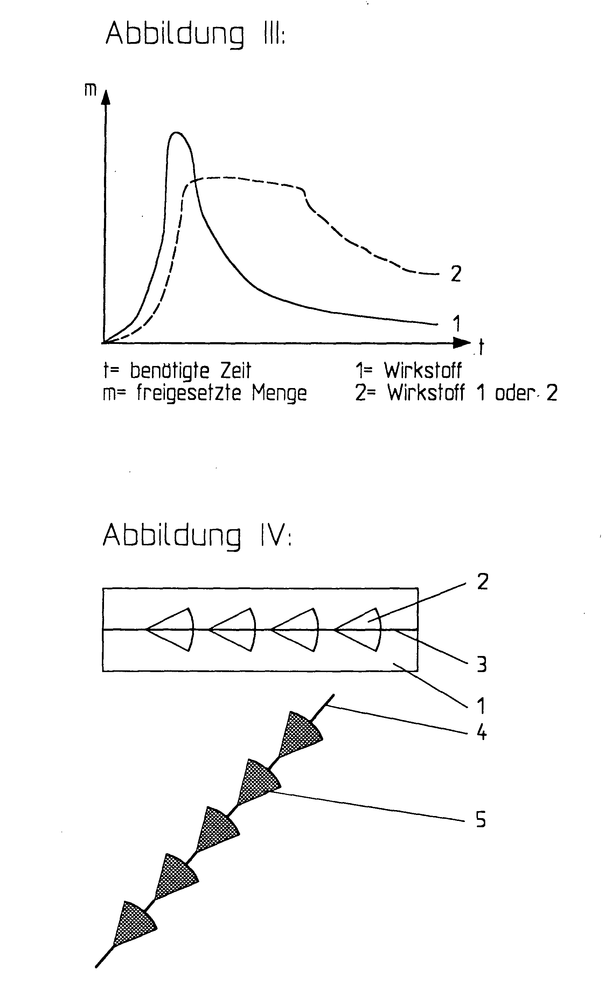

## Patentansprüche

1. Verfahren zur Herstellung eines antibakteriellen Wirkstoffes,
**dadurch gekennzeichnet,**
**daß** ein Mono- oder Copräzipitat in kristalliner und/oder amorpher Form durch Lösung mit anschließender gesteuerten Kristallisation, der Umkristallisation, von einer Lösung eines oder mehreren Wirkstoffen hergestellt wird,
**daß** das Lösungsmittel ganz oder teilweise entfernt wird, und
**daß** der Wirkstoff oder die Wirkstoffe so geformt werden, z.B. durch kornselektrierendes Mahlverfahren, daß eine Korngrößenverteilung, die einer gestauchten gauß'schen Glockenkurve mit abgeflachtem Plateau folgt, erhalten wird, sodaß ein der Freisetzungszeit, Freisetzungsform und Freisetzungsmenge optimaler, vorbestimmbarer, antibakterieller Wirkstoff entsteht.

2. Verfahren nach Anspruch 1, wobei der entstandene Wirkstoff zu Körnern mit vorzugsweise trapezförmiger Korngrößenverteilung zerkleinert wird.

3. Verfahren nach Anspruch 1, wobei der Wirkstoff zu Formkörpern mit definierter Geometrie zerschnitten wird.

4. Verfahren nach Anspruch 1, wobei die Umkristallisation in Formen erfolgt, die der Applikationsform entsprechen und keine weitere Formgebung erfolgt.

5. Verfahren nach einem der Ansprüche 1-4, wobei der entstandene Wirkstoff mit einem weiteren Wirkstoff umhüllt wird.

6. Verfahren nach Anspruch 5, wobei eine weitere Wirkstoffschicht aufgebracht wird.

7. Verfahren nach einem der Ansprüche 1-6, wobei die Wirkstofflösung mit einem Füllstoff versehen wird.

8. Verfahren nach einem der Ansprüche 1-7, wobei die eingesetzten Wirkstoffe Aminoglykoside, Cepholosporine, Penizilline oder artverwandte Wirkstoffe sind, bevorzugt jedoch, Gentamycin, Clindamycin, Erythromycin oder Vancomycin.

9. Verfahren nach einem der Ansprüche 1-8, wobei zumindest ein eingesetzter Füllstoff in chemisch gefällter Form oder als Keramik vorliegen und entweder resorbierbar oder nichtresorbierbar ist.

10. Verfahren nach einem der Ansprüche 7-9, wobei zumindest ein eingesetzter Füllstoff vorzugsweise aus Calciumphosphate, Aluminiumoxide, Zirkonoxide oder ähnlichem, bzw. aus Mischungen desselben besteht.

11. Verfahren nach einem der Ansprüche 7-8, wobei zumindest ein Füllstoff Gips ist.

12. Verfahren nach Anspruch 11, wobei eine Aushärtung des Gipses in vitro erfolgt.

13. Verfahren nach einem der Ansprüche 1-12, wobei die Wirkstofform als Stange, Plättchen, Kette, Kasten oder anderen geometrischen Formen, einzeln oder im Verbund vorliegen.

14. Verwendung eines im Herstellungsverfahren gemäss einem der Ansprüche 1-13 hergestellten Materials als Zuschlagstoff für medizinische Knochenzemente.

15. Verwendung eines im Herstellungsverfahren gemäss einem der Ansprüche 1-13 hergestellten Materials als Zuschlagstoff für Formkörper für die medizinische Anwendung.

16. Verwendung des Materials nach Anspruch 15 in der äußeren (nicht in vivo) medizinischen Anwendung.

17. Verwendung eines im Herstellungsverfahren gemäss einem der Ansprüche 1-13 hergestellten Materials als Zuschlagstoff für medizinische Wachse, Kleber, Siegel oder andere Trägermateriale.

18. Verwendung eines im Herstellungsverfahren gemäss einem der Ansprüche 1-13 hergestellten Materials zur Herstellung von Streukorn.

19. Verwendung eines im Herstellungsverfahren gemäss einem der Ansprüche 1-13 hergestellten Materials als Beschichtungsmaterial für Implantate oder zur Einbringung in dafür vorgesehene Kavitäten in den Implantaten.

## Claims

1. Method for producing an antibacterial active substance,
**characterized in**
**that** a mono- or co-precipitate in crystalline and/or amorphous form is produced by dissolution with subsequent controlled crystallization, recrystallization, of a solution of one or more active substances,
**that** the dissolvent is completely or partially removed, and
**that** the active substance or substances is/are shaped, i.e. in a grain or particle selecting grinding method, such that a grain or particle size distribution, which follows a battered gaussean bell curve with a flattened plateau, is obtained, so that a predetermined antibacterial active substance with optimal time, shape and amount of release is produced.

2. Method according to claim 1, wherein the produced active substance is crushed to grains or particles with preferably trapezoidal grain or particle size distribution.

3. Method according to claim 1, wherein the active substance is cut into shaped bodies with defined geometry.

4. Method according to claim 1, wherein the recrystallization is carried through into shapes corresponding with the shape of application and no further shaping is carried through.

5. Method according to any of claims 1-4, wherein the produced active substance is coated with an additional active substance.

6. Method according to claim 5, wherein an additional layer of active substance is applied.

7. Method according to any of claims 1-6, wherein a filler agent is added to the solution of active substance or substances.

8. Method according to any of claims 1-7, wherein the active substances used are aminoglycosides, cepholosporines, penicillines or related active substances, preferably however, gentamycin, clindamycin, erythromycin or vancomycin.

9. Method according to any of claims 1-8, wherein at least one added filler agent is present in chemically precipitated form or as a ceramic and is either resorbable or non-resorbable.

10. Method according to any of claims 7-9, wherein at least one added filler agent preferably consists of calcium phosphates, aluminium oxides, zirconium oxides or similar, respectively of mixtures thereof.

11. Method according to any of claims 7-8, wherein at least one filler agent is gypsum.

12. Method according to claim 11, wherein hardening of the gypsum in vitro is carried through.

13. Method according to any of claims 1-12, wherein the shape of the active substance is present as a rod, plate, chain, box or other geometric shapes, separate or in combination.

14. Use of a material produced according to the method of any of claims 1-13 as an additive for medical bone cements.

15. Use of a material produced according to the method of any of claims 1-13 as an additive for shaped bodies for medical use.

16. Use of the material according to claim 15 in the exterior (not in vivo) medical use.

17. Use of a material produced according to the method of any of claims 1-13 as an additive for medical waxes, adhesives, sealing agents or other carrier materials.

18. Use of a material produced according to the method of any of claims 1-13 for production of scatter grains or particles.

19. Use of a material produced according to the method of any of claims 1-13 as a coating material for implants or for location in cavities provided therefor in the implants.

## Revendications

1. Procédé pour préparer une substance active antibactérienne, **caractérisé en ce que**
on prépare un mono ou co-précipité à l'état cristallin et/ou amorphe par dissolution avec cristallisation contrôlée ultérieure, recristallisation, d'une solution d'une substance ou de plusieurs substances actives,
le dissolvant est entièrement ou partiellement éliminé, et
la substance ou les substances actives est/sont mise(s) en forme, c-à-d. par une méthode de broyage en sélectionnant des grains ou des particules, tels qu'on obtient une répartition des dimensions de grains ou particules, qui suit une courbe de Gausse en forme de cloche endommagée avec un plateau aplati, de manière à ce qu'on prépare une substance active antibactérienne prédéterminée avec un temps, une forme et une quantité de libération optimale.

2. Procédé selon la revendication 1, dans lequel la substance active produite est écrasée pour former des grains ou des particules avec de préférence une répartition trapézoïdale des dimensions des particules ou des grains.

3. Procédé selon la revendication 1, dans lequel la substance active est coupée sous forme de corps à géométrie définie.

4. Procédé selon la revendication 1, dans lequel la recristallisation est mise en oeuvre dans des formes correspondant à la forme de l'application et aucune mise en forme supplémentaire n'est mise en oeuvre.

5. Procédé selon l'une quelconque des revendications 1 à 4, dans lequel la substance active préparée est recouverte d'une substance active supplémentaire.

6. Procédé selon la revendication 5, dans lequel on applique une couche supplémentaire d'une substance active.

7. Procédé selon l'une quelconque des revendications 1 à 6, dans lequel on ajoute un agent de remplissage à la solution de la substance ou des substances active(s).

8. Procédé selon l'une quelconque des revendications 1 à 7, dans lequel les substances actives utilisées sont des aminoglycosides, cépholosporines, pénicillines ou des substances actives apparentées, de préférence toutefois, la gentamycine, clindamycine, l'érythromycine ou la vancomycine.

9. Procédé selon l'une quelconque des revendications 1 à 8, dans lequel au moins un agent de remplissage ajouté est présent sous forme d'un précipité chimique ou sous forme d'une céramique et peut se résorber ou non.

10. Procédé selon l'une quelconque des revendications 7 à 9, dans lequel au moins un agent de remplissage ajouté consiste de préférence en des phosphates de calcium, oxydes d'aluminium, oxydes de zirconium ou similaires, respectivement des mélanges de ceux-ci.

11. Procédé selon l'une quelconque des revendications 7 à 8, dans lequel au moins un agent de remplissage est le gypse.

12. Procédé selon la revendication 11, dans lequel le durcissement du gypse est mis en oeuvre in vitro.

13. Procédé selon l'une quelconque des revendications 1 à 12, dans lequel la forme de la substance active est présente en tant qu'une tige, plaque, chaîne, boîte ou autres formes géométriques, séparées ou en combinaison.

14. Utilisation d'une matière préparée suivant le procédé selon l'une quelconque des revendications 1 à 13 sous forme d'un additif pour des céments médicaux d'os.

15. Utilisation d'une matière préparée suivant le procédé selon l'une quelconque des revendications 1 à 13 sous forme d'un additif pour des corps pour une utilisation médicale.

16. Utilisation de la matière selon la revendication 15 dans une utilisation médicale externe (non in vivo).

17. Utilisation d'une matière préparée suivant le procédé selon l'une quelconque des revendications 1 à 13 sous forme d'un additif pour des résines, adhésifs, agents médicaux de scellage ou autres matières de support.

18. Utilisation d'une matière préparée suivant le procédé selon l'une quelconque des revendications 1 à 13 pour la production d'un pulvérisateur de grains ou de particules.

19. Utilisation d'une matière préparée suivant le procédé selon l'une quelconque des revendications 1 à 13 sous forme d'une matière de revêtement pour des implants ou pour une localisation dans des cavités prévues dans ce but dans les implants.
